# EUROPEAN PATENT APPLICATION

(11) **EP 4 454 554 A1**
(43) Date of publication of application: **30.10.2024**
(21) Application number: 23169521.4
(22) Date of filing: 24.04.2023
(51) Int. Cl.: A61B 5/08, A61B 5/113, A61B 5/00

(54) **ESTIMATING RESPIRATION RATE USING A DIMENSIONALITY REDUCTION ALGORITHM**

(71) Applicant: Koninklijke Philips N.V., 5656 AG Eindhoven (NL)
(72) Inventor: FRANCK, Christoph Florian, Eindhoven (NL)
(74) Representative: Philips Intellectual Property & Standards

(57) **Abstract**

Proposed concepts thus aim to provide schemes, solutions, concept, designs, methods and systems pertaining to estimating respiration rate of a subject. This can be achieved by processing a plurality of input signals indicative of detected thorax movement of a subject with a dimensionality reduction algorithm. Proposed concepts thus aim to process multiple different input signals, each indicative of detected thorax movement of the subject, with a dimensionality reduction algorithm, thereby generating multiple output channels, wherein each output channels represents a respective component of the plurality of input signals. For example, it is likely that one of the output channels comprises a component corresponding to the respiration rate of the subject, and the remaining output channels contain noise or other irrelevant components such as interference artefacts.

## Description

### FIELD OF THE INVENTION

This invention relates to the field of estimating a respiration rate, and in particular to the field of estimating the respiration rate of a subject.

### BACKGROUND OF THE INVENTION

The respiration rate of a patient or subject is an important clinical parameter. Changes in the respiration rate typically occur ahead of degradation in the patient's state, making respiration rate a useful predictor of adverse events and a component of early warning score algorithms for patient state assessment.

A common way of detecting the respiratory activity of a patient is based on measuring the electric impedance across the patient's thorax. This impedance undergoes small changes when the thorax moves and changes (internal and external) geometry due to the patient inhaling and exhaling. This measurement is usually integrated into electrocardiographs (ECG), as the electrodes used for recording the electrocardiogram can be used for the thoracic impedance measurement as well. A thoracic impedance measurement is done by applying an excitation signal - with a small voltage and a frequency in the range of tens of kilohertz - between two electrodes attached to the patient's torso or limbs. A recorder measures the change of the voltage drop between the electrodes that is caused by the change of impedance across the thorax.

The change in impedance is recorded as a function of time, and commonly presented to the user in the form of a graph or wave. Software in recorders or patient monitors usually derive a respiration rate numeric from this data and present the numeric on the display as well.

Current ECG/respiration recorders usually measure the impedance between a pair of electrodes using a single frequency for the excitation signal. Some models allow the user to choose which of the ECG electrodes are used for the respiration measurements, for example, left-leg/right-arm pair or left-arm/right-arm pair.

However, current users usually have low confidence in the automated respiration rate measurement of patient monitors. The measurements are susceptible to artefacts when the patient moves, and to electrical/electromagnetic interference from external sources. These issues are in contrast to the importance of respiration rate as a parameter for assessing current patient state and predicting future changes in patient state.

Current respiration measurements integrated into ECG/respiration recorders typically use only a single frequency for the excitation signal, and measure thoracic impedance across a single pair of electrodes. As already mentioned, some models allow the user to select which pair of electrodes are used or to adapt the measurement to different breathing movement patterns. As the breathing movements of patients may change, e.g. by emphasizing the use of diaphragmatic muscles or intercostal muscles when breathing, a one-time setting of the electrode pair may not stay optimal over time. The user is therefore required to adjust the electrode pair or accept a degradation of the accuracy/availability of the respiration rate measurements.

### SUMMARY OF THE INVENTION

The invention is defined by the claims.

According to examples in accordance with an aspect of the invention, there is provided a method for estimating respiration rate of a subject. The method comprises: obtaining a plurality of input signals indicative of detected thorax movement of the subject, each of the plurality of input signals representing thorax movement detected via a respective detection arrangement; processing the plurality of input signals with a dimensionality reduction algorithm to generate a plurality of output channels, wherein each channel represents a respective component of the plurality of input signals; and estimating a respiration rate of the subject based on at least one of the plurality of output channels.

Proposed concepts thus aim to provide schemes, solutions, concept, designs, methods and systems pertaining to estimating respiration rate of a subject. In particular, embodiments aim to provide a method for estimating respiration rate of a subject by using a dimensionality reduction algorithm to process a plurality of input signals, each representing thorax movement, thereby providing a more reliable and accurate estimated respiration rate based on at least one of the output channels of the dimensionality reduction algorithm.

In other words, it is proposed that by using a dimensionality reduction algorithm, multiple different input signals, each indicative of detected thorax movement of the subject, can be processed thereby generating multiple output channels, wherein each output channel represents a respective component of the plurality of input signals. For example, it is likely that one of the output channels comprises a component corresponding to the respiration rate of the subject, and the remaining output channels contain noise or other irrelevant components such as interference artefacts.

By using a dimensionality reduction algorithm, multiple input signals can be processed to produce an output channel containing the respiration signal of the subject with less random noise than each individual input signal. This is because the respiration signal component is correlated across the input signals but random noise is not. Other irrelevant components can also be separated out of the input signals by using a dimensionality reduction algorithm, such as a component related to subject movements, or, if using impedance measurements for example, electrical interference from nearby equipment.

In a simple example, two input signals can be obtained and processed thereby producing two output channels, wherein one represents a respiration signal component, and the other represents a noise component. A respiration signal with an enhanced signal-to-noise (SNR) ratio is thereby provided and a more accurate and reliable respiration rate of the subject can be estimated. Using a dimensionality reduction algorithm provides a better use of the available information provided in the input signals, in terms of producing a respiration signal with low SNR. This is in contrast to simple selection algorithms such as merely picking the input signal with the largest variation over time, or merely averaging the input signals.

The inventors have realised that using a dimensionality reduction algorithm to process multiple input signals, each representing thorax movement detected via a respective detection arrangement, provides a more robust, accurate, and reliable method of estimating respiration rate of a subject. A respective detection arrangement may comprise a respective detection technique, approach, position, location, frequency, pathway, vector, time of detection, etc. What is important is that the detection arrangement of each respective input signal differs in some way to one another, such that there are provided input signals that differ from one another, but each comprise a common respiration signal component. In other words, the input signals may all be obtained with the same hardware, simply varying in one or more parameters of detection, such as time of application, frequency of impedance measurement, and/or location on the subject's body, for example. Alternatively, the input signals may each be obtained with different hardware, such as, for example, multiple electrode pairs placed in different locations on the subject's body and using different excitation frequencies. Pairs of electrodes may be referred to as a vector, respiration vector, or pathway.

This invention may be of particular use in the case of thoracic impedance measurements, in which electrical interference from nearby equipment in a certain frequency range or subject movement may increase noise in a signal to such a degree that a respiration rate estimate becomes impossible or very unreliable. By obtaining multiple thoracic impedance signals, the common respiration signal component between them can be found using a dimensionality reduction algorithm, such as a principle component analysis (PCA) algorithm or an independent component analysis (ICA) algorithm. The resulting output channel containing the common respiration signal component can then be utilised to estimate a respiration rate of the subject and, optionally, other relevant respiration parameters. In summary, in the case of thoracic impedance measurements, this invention is meant to improve the accuracy, reliability, and availability of respiration rate measurements by reducing artefacts. The artefacts may be caused by electric/electromagnetic interference and/or patient movements, and by using a dimensionality reduction algorithm the respiratory movements of the patients may be reproduced optimally in a derived respiration wave. The invention also frees the clinical user from the task of setting and adjusting an electrode pair ("lead vector") used for the thoracic impedance measurements.

Ultimately, an improved method for estimating respiration rate of a subject is provided.

In some embodiments, the plurality of input signals may comprise thoracic impedance signals, each of the plurality of input signals representing thorax impedance detected via a respective impedance detection arrangement. Measuring the electric impedance across a subject's thorax is a common way of detecting the respiratory activity of a patient.

In some embodiments, the plurality of input signals may be obtained for respective different excitation signal frequencies, and preferably wherein the different excitation signal frequencies comprise different bands of frequencies respectively. If there is any electrical interference at a certain frequency, another input signal at a different frequency can then be used by the dimensionality reduction algorithm to, in essence, filter the interference component out. Using different bands of frequencies allows for excitation signals to be used that are not sinusoidal.

In some embodiments, obtaining the plurality of input signals may comprise using at least one pair of electrodes to apply at least one excitation signal frequency to the subject. A pair of electrodes is a common way of measuring thoracic impedance, but can also be used for other obtaining other types of signals indicative of detected thorax movement of the subject. Electrodes provide a relatively cheap, efficient, and effective way of applying excitation signals and measuring thorax movement.

In some embodiments, the plurality of input signals may represent thorax movement of the subject detected simultaneously. By having the input signals represent simultaneously detected thorax movement, the respiration signal between the input signals will be strongly correlated, and thus the dimensionality reduction algorithm will be more likely to be able to reliably and accurately extract the respiration component from them.

In some embodiments, obtaining the plurality of input signals may comprise using at least one pair of electrodes to simultaneously apply at least two different excitation signal frequencies to the subject, and to detect thoracic impedance signals for each of the different excitation signal frequencies respectively. By using at least two different excitation signal frequencies, if there is electrical interference at one frequency, the dimensionality reduction algorithm may still be able to extract a relatively clean respiration signal from the input signals. It essentially does this by finding the common respiration signal component present in both the input signals.

In some embodiments, the plurality of input signals may represent thorax movement of the subject detected at different non-overlapping time periods respectively. This provides more flexibility to the method, wherein input signals may not need to be detected simultaneously. For example, the input signals may be time-multiplexed. Further, in the case of applying two different excitation frequencies with a single pair of electrodes, if the excitation frequencies are applied non-simultaneously, each impedance input signal may be acquired at a respectively higher current than if both were acquired simultaneously. This may lead to a better SNR for each input signal.

In some embodiments, obtaining the plurality of input signals may comprise using at least one pair of electrodes to apply at least two different excitation signal frequencies to the subject at different non-overlapping time periods, and to detect thoracic impedance signals for each of the different excitation signal frequencies respectively.

In some embodiments, the dimensionality reduction algorithm may comprise at least one of a principal component analysis algorithm and an independent component analysis algorithm. Principle component analysis (PCA) can process a set of input signals into uncorrelated output channels, and independent component analysis (ICA) can process a set of input signals to decompose them into statistically independent output channels. ICA algorithms often contain PCA as a first (pre-processing) step or integrate a PCA component into their coefficient update algorithm.

In some embodiments, estimating a respiration rate of the subject based on at least one of the plurality of output channels may comprise: analysing the plurality of output channels to determine which output channel is more probable to comprise information corresponding to the respiration rate of the subject; and estimating a respiration rate of the subject based on the determined output channel. After the dimensionality reduction algorithm has processed the input signals, it is likely only one of the output channels will contain a respiration signal component, and so by analysing the output channels to determine which is most likely to comprise information corresponding to the respiration rate of the subject, the respiration rate can subsequently be estimated based on that determined output channel.

In some embodiments, analysing the plurality of output channels to determine which output channel is more probable to comprise information corresponding to the respiration rate of the subject may comprise analysing at least one of: the variance, skewness, kurtosis, metrics of entropy, dominant frequency, and total variation of the plurality of output channels. These metrics may aid in the determining of which output channel is most likely to comprise the respiration signal component. For example, the total variation of the respiration signal component is likely to be low.

In some embodiments, obtaining the plurality of input signals may comprise using at least one of: at least one strain gauge attached to the thorax, and at least one accelerometer attached to the thorax. These devices provide relatively simple ways of obtaining input signals indicative of detected thorax movement of the subject.

In some embodiments, the at least one pair of electrodes may be attached to the subject's torso or limbs. This may provide an easy way for electrodes to provide signals indicative of thorax movement.

In some embodiments, the at least one pair of electrodes may be attached to at least one of: the subject's right arm and left leg, right arm and left arm, right arm and right leg, left arm and right leg, and left arm and left leg. Each pair of locations is referred to as a vector or pathway.

In some embodiments, analysing the plurality of output channels to determine which output channel is more probable to comprise information corresponding to the respiration rate of the subject comprises analysing the plurality of output channels to determine which output channel is more probable to comprise an impedance signal corresponding to the subject's respiration.

In some embodiments, the method may further comprise time-multiplexing the plurality of input signals to generate a plurality of time-multiplexed input signals.

In some embodiments, the method may further comprise performing at least one of: demodulation, low-pass filtering and sampling rate reduction on the plurality of input signals.

In some embodiments, the method may further comprise generating a display control signal, the display control signal describing the estimated respiration rate of the subject, and optionally, the determined output channel.

According to another aspect of the invention, there is provided a computer program comprising code means for implementing the method of any herein disclosed method when said program is run on a processing system.

According to another aspect of the invention, there is provided a system for estimating respiration rate of a subject. The system comprising an input interface configured to: obtain a plurality of input signals indicative of detected thorax movement of the subject, each of the plurality of input signals representing thorax movement detected via a respective detection arrangement; and a processor configured to: process the plurality of input signals with a dimensionality reduction algorithm to generate a plurality of output channels, wherein each output channel represents a respective component of the plurality of input signals; and
estimate a respiration rate of the subject based on at least one of the plurality of output channels.

In some embodiments, the system may further comprise at least one pair of electrodes configured to generate the plurality of input signals, wherein the plurality of input signals are thoracic impedance signals.

Thus, there may be proposed concepts for estimating respiration rate of a subject, and this may be done based on processing a plurality of input signals indicative of detected thorax movement of a subject with a dimensionality reduction algorithm.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for estimating respiration rate of a subject according to a proposed embodiment;
Figs. 2A and 2B are diagrams each showing one of the plurality of input signals respectively, according to a proposed embodiment;
Figs. 2C and 2D are diagrams each showing one of the plurality of output channels respectively, according to a proposed embodiment;
Fig. 3 is a flow diagram of a method for estimating respiration rate of a subject from two thoracic impedance input signals according to a proposed embodiment;
Fig. 4 is a flow diagram of a method for estimating respiration rate of a subject from a plurality of non-simultaneous input signals according to a proposed embodiment;
Fig. 5 is a simplified block diagram of a system for estimating respiration rate of a subject according to a proposed embodiment; and
Fig. 6 illustrates an example of a computer within which one or more parts of an embodiment may be employed.

### DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to estimating respiration rate of a subject. According to proposed concepts, a number of possible solutions may be implemented separately or jointly. That is, although these possible solutions may be described below separately, two or more of these possible solutions may be implemented in one combination or another.

Embodiments of the invention aim to provide a method for estimating respiration rate of a subject. This can be achieved by processing a plurality of input signals indicative of detected thorax movement of a subject with a dimensionality reduction algorithm.

Proposed concepts thus aim to process multiple input signals, each indicative of detected thorax movement of the subject, with a dimensionality reduction algorithm, thereby generating multiple output channels. Each output channel represents a respective component of the plurality of input signals. For example, it is likely that one of the output channels comprises a component corresponding to the respiration rate of the subject, and the remaining output channels contain noise or other irrelevant components such as interference artefacts.

In other words, by using a dimensionality reduction algorithm, multiple input signals can be processed to produce an output channel containing the respiration signal of the subject with less random noise than any one individual input signal. This is because the respiration signal component is correlated across the input signals but random noise is not. Other irrelevant components can also be separated out of the input signals by using a dimensionality reduction algorithm, such as a component related to subject movement, or, if using impedance measurements for example, electrical interference from nearby equipment.

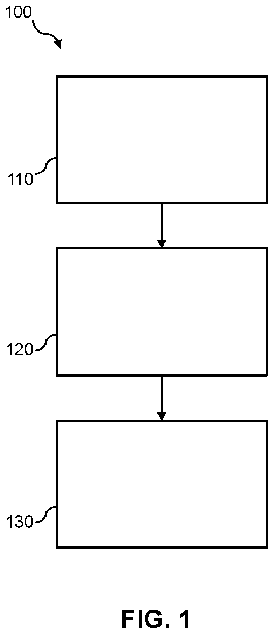

Referring now to Fig. 1, there is depicted a simplified flow diagram of a method 100 for estimating respiration rate of a subject according to a proposed embodiment.

The method 100 begins with the step 110 of obtaining a plurality of input signals indicative of detected thorax movement of the subject, each of the plurality of input signals representing thorax movement detected via a respective detection arrangement. In some embodiments, the plurality of input signals may be obtained using at least one pair of electrodes to apply at least one excitation signal frequency to the subject. A pair of electrodes is a common way of measuring thoracic impedance, but can also be used for other obtaining other types of signals indicative of detected thorax movement of the subject. Electrodes provide a relatively cheap, efficient, and effective way of applying excitation signals and measuring thorax movement. The pair of electrodes may, in some embodiments, be attached to the subject's torso or limbs. This may provide an easy way for electrodes to provide signals indicative of thorax movement. For instance, the at least one pair of electrodes may be attached to at least one of: the subject's right arm and left leg, right arm and left arm, right arm and right leg, left arm and right leg, and left arm and left leg. In some embodiments, multiple pairs of electrodes attached to the subject in different locations may be used to each obtain at least one of the plurality of input signals respectively. In this way, the effect of any interference in the signals due to the subject's movement may be, in essence, filtered out of the input signals during processing with the dimensionality reduction algorithm. This is because interference from the subject's movement will be uncorrelated across the different locations of the electrode pairs, i.e. across the different vectors.

It should be noted, electrodes are not required to obtain the plurality of input signals. Other suitable devices may be used to obtain the plurality of input signals indicative of detected thorax movement. For instance, at least one strain gauge or accelerometer attached to the thorax may be used. These devices provide relatively simple ways of obtaining input signals indicative of detected thorax movement of the subject.

A respective detection arrangement may comprise a respective detection technique, approach, position, location, frequency, pathway, vector, time of detection, etc. What is important is that the detection arrangement of each respective input signal differs in some way to one another, such that there are provided input signals that differ from one another, but each comprise a common respiration signal component. In other words, the input signals may all be obtained with the same hardware, simply varying in one or more parameters of detection, such as time of application, frequency of impedance measurement, or location on the subject's body, for example. Alternatively, the input signals may each be obtained with different hardware, such as, for example, multiple electrode pairs placed in different locations on the subject's body.

In this embodiment, the plurality of input signals represents thorax movement of the subject detected simultaneously. By having the input signals represent simultaneously detected thorax movement, the respiration signal component will be strong correlated between the input signals, and thus the dimensionality reduction algorithm will be able to reliably and accurately extract the respiration component from them. The greater the number of input signals the more robust the respiration rate estimate will be against interference any that affects only a subset of the input signals.

Under normal conditions, all input signals should contain a component corresponding to the breathing movements of the subject (which should be highly correlated in all the input signals), and a component corresponding to noise (which is usually weakly correlated or uncorrelated). PCA, for example, can then be used to find a linear combination of the input signals which contains the correlated respiration component in all (or a majority) of the input signals.

In step 120, the plurality of input signals is processed with a dimensionality reduction algorithm to generate a plurality of output channels, wherein each output channel represents a respective component of the plurality of input signals. In this embodiment, the dimensionality reduction algorithm comprises an independent component analysis (ICA) algorithm. In other embodiments, however, the dimensionality reduction algorithm may comprise a principal component analysis (PCA) algorithm. ICA can process a set of input signals to decompose them into statistically independent output channels. ICA algorithms often contain PCA as a first (pre-processing) step or integrate a PCA component into their coefficient update algorithm.

PCA takes N input signals (otherwise referred to as input channels) and decomposes the signals into N output channels, y(t), that contain uncorrelated (principal) components. PCA also provides the coefficients for the linear combinations that generate each output channel from the set of input signals. Examining the coefficients can show whether one particular output component occurs in most, or all, of the input channels, or whether it is limited to a small subset of the input signals, even to one individual input signal.

Similar to PCA, ICA also provides the coefficients for the linear combinations that form the output channels from the input channels. Using ICA is especially beneficial if more than one of the expected signal sources does not have a normal distribution, i.e. if interference or artefacts are expected that do not exhibit a normal distribution.

In some embodiments, before step 120, the method 100 may further comprise performing at least one of: demodulation, low-pass filtering and sampling rate reduction on the plurality of input signals.

In step 130, a respiration rate of the subject is estimated based on at least one of the plurality of output channels. There are known standard techniques for deriving a respiration rate from a respiration signal or signal component.

In some embodiments, the method then further comprises generating a display control signal, the display control signal describing the estimated respiration rate of the subject. In this way, the estimated respiration rate may be provided to a clinical user and/or the subject via a display device.

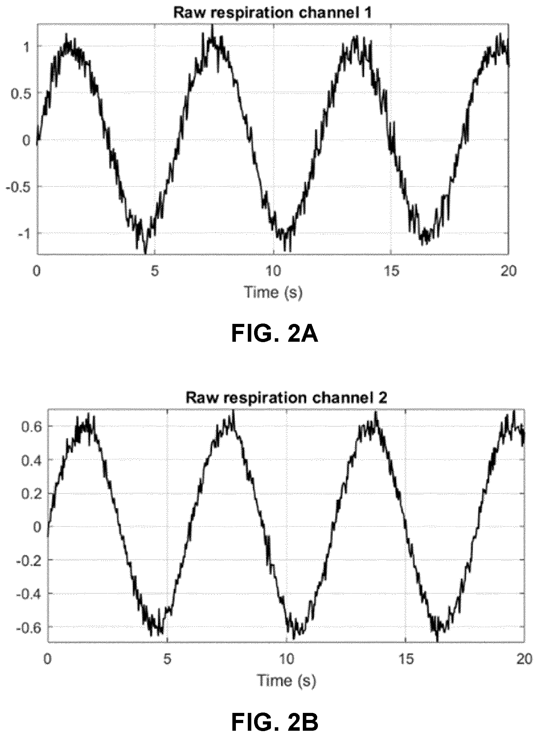

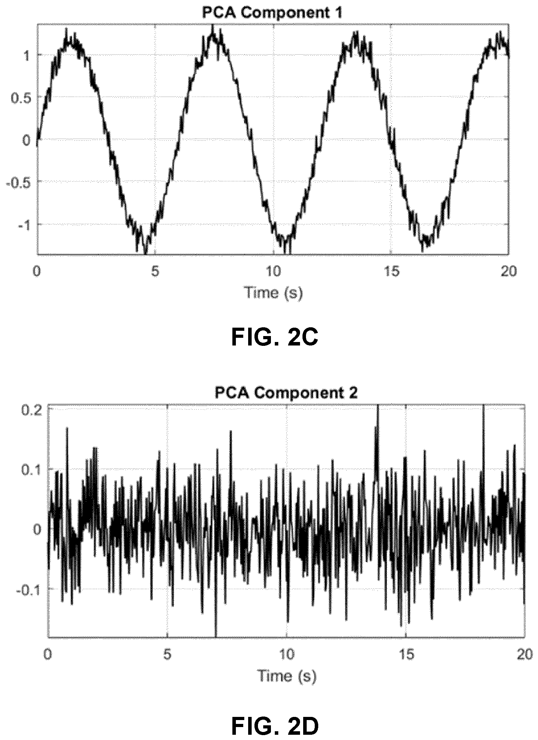

Referring now to Figs. 2A and 2B, there are depicted diagrams showing one of the plurality of input signals respectively, according to a proposed embodiment. As can be seen, each input signal comprises a relatively noisy respiration signal. In this case, the input signals are thoracic impedance signals. Processing the two input signals shown in Figs. 2A and 2B with a PCA algorithm generates two output channels, shown in Figs. 2C and 2D respectively. As can be seen, a respiration signal with a better SNR than either of the two raw input signals (Figs. 2A and 2B) is provided in the first output channel shown in Fig. 2C. An output channel comprising a noise component is provided in Fig. 2D. A more reliable and accurate respiration rate can therefore be estimated using the output channel seen in Fig. 2C, as compared to either one of the input signals (Figs. 2A and 2B).

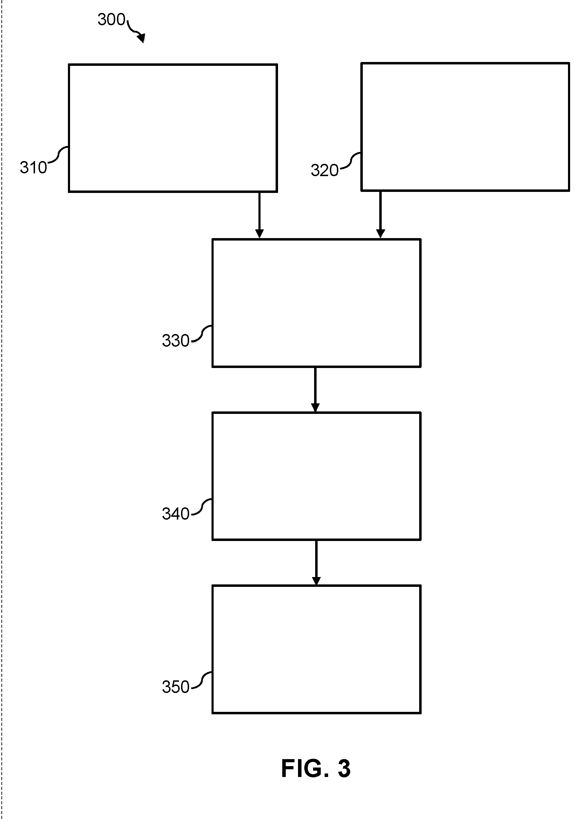

Referring now to Fig. 3, there is depicted a flow diagram of a method 300 for estimating respiration rate of a subject from two thoracic impedance input signals according to a proposed embodiment.

In step 310, a first thoracic impedance signal is obtained, indicative of detected thorax movement of the subject. In step 320, a second thoracic impedance signal is obtained, indicative of detected thorax movement of the subject. Measuring the electric impedance across a subject's thorax is a common way of detecting the respiratory activity of a patient.

Thoracic impedance is measured by generating an alternating current (AC) excitation signal and applying the excitation voltage across internal resistors and a corresponding electrode pair. As the geometry of the subject's thorax changes during respiratory activity, the impedance in each input signal will change. The changes caused by respiratory activity will be strongly correlated across the input signals, while noise, effects of interference, and some forms of movement artefacts will be not correlated or only occur in a subset of the input signals. The resulting AC voltage across the electrode pair can be measured by either sampling it directly with an analog to digital converter (ADC) and demodulating in software, or by demodulating in hardware and sampling the resulting low-frequency voltage signal with an ADC. Both approaches result in digitally sampled impedance signals.

The sampled impedance signals can then be used as inputs, i.e. input signals, for a dimensionality reduction algorithm, such as PCA or, preferably, ICA. If ICA is used, however, the amplitude of the respiration signal is likely to have to be reconstructed, as this information is not retained by many ICA algorithm implementations.

Impedance changes due to respiratory activity produce a roughly sinusoidal signal component in the input impedance signal channels. The values of a sampled sinusoidal signal have a distinctive statistical distribution that is different from a normal (Gaussian) distribution. While PCA can derive no benefit from this prior information, ICA can decompose multiple input signals into output channels that are statistically independent, which is preferable to the merely uncorrelated outputs produced by PCA.

In this embodiment, the first and second thoracic impedance signals are obtained for respective different excitation signal frequencies. If there is any electrical interference at a certain frequency, another input signal at a different frequency can then be used by the dimensionality reduction algorithm to, in essence, filter the interference component out and extract a relatively clean respiration component. In some embodiments, the different excitation signal frequencies comprise different bands of frequencies respectively. Using different bands of frequencies allows for the use of excitation signals which are not sinusoidal. Each band may specify a range, for example, 40.0 kHz to 40.1 kHz. The excitation signal would then be a signal that occupies some or all of this range, for example: a frequency-modulated or amplitude-modulated signal with a centre frequency of 40.05kHz; a superposition of sine waves of different frequencies in the frame from 40.0kHz to 40.1kHz; or random noise that is bandlimited to this frequency range.

In typical current methods of respiration rate determination using thoracic impedance measurements, only a single excitation signal frequency is used. If there is interference at this particular frequency from devices in the vicinity or in contact with the patient, the respiration rate measurement may become inaccurate or unavailable as long as the interfering signal is present. By using two or more different excitation signal frequencies, the respiration measurement is made robust against narrow-band interference. Further, as thoracic impedance measurements only measure small changes in impedance occurring in series to a large, constant impedance, the SNR of this measurement is often an issue, even when no specific interference or movement artefacts are present. After processing the input signals with either a PCA or ICA algorithm, the component of the impedance variation that is attributable to respiratory movements will be present in one of the output channels, while the other output channel(s) will contain noise and/or other undesired components. The output channel containing the respiration signal will have less random noise than each individual input signal due to the weighted average effect of dimensionality reductions algorithms, since the random noise component is uncorrelated across the input signals.

In this embodiment, the first and second thoracic impedance signals are obtained using one pair of electrodes to simultaneously apply two different excitation signal frequencies to the subject, and to detect thoracic impedance signals for each of the two different excitation signal frequencies respectively. By using at least two different excitations signal frequencies, if there is electrical interference at one frequency, the dimensionality reduction algorithm may still be able to extract a relatively clean respiration signal from the input signals, by finding the common respiration signal component present in both the input signals. Hardware is therefore required capable of multiple simultaneous thoracic impedance measurement channels.

In other words, if one of the plurality of impedance input signals contains a large interference component and the other(s) does not, for instance, due to narrow-band interference, then the respiration wave signal will appear in one of the output channels and the interference will appear in another output channel.

Patient movement also often causes artefacts in the thoracic impedance measurements and can alter the derived estimated respiration rate. Using only one impedance measurement channel, as is currently typical, it is very difficult to distinguish between movement artefacts and respiratory activity. Since the impedance changes due to patient movements are usually uncorrelated with the breathing movement, however, using a dimensionality reduction algorithm will also increase the robustness of obtaining a clean respiration measurement signal regardless of patient movement artefacts in the input signals.

Further, typical current respiration rate determination methods by thoracic impedance measurements use only a single electrode pair for the measurement, which often has to be configured by the user. If the subject's breathing motion pattern does not cause sufficiently large impedance changes across the single vector, the respiration rate measurement may become inaccurate or unavailable. The subject may not know about the option to change the respiration vector or be hesitant to use this feature due to lack of experience. However, embodiments of this invention provide a method with multiple respiration vectors measured, either simultaneously or non-simultaneously. These can then be processed by a dimensionality reduction algorithm, and therefore the user does not need to choose or change the respiration measurement vector themselves, rather, the dimensionality reduction algorithm will automatically find the optimal linear combination of the input signals, even when the patient's breathing motion pattern changes.

In some embodiments, a plurality of electrode pairs may be used, each electrode pair having a different excitation signal frequency respectively. Embodiments with more than one respiration vector have the additional technical effect of a dimensionality reduction algorithm automatically finding the linear combination of the input signals which gives the best/largest representation of the impedance change due to movements. Therefore, manual, user-driven selection of the best respiration vector is not necessary. Impedance changes due to subject movement, which create artefacts in the respiration wave, appear in a different output channel if they are uncorrelated with the respiration signal. This effectively removes the effect of movement from the respiration wave and the derived estimated respiration rate.

In some embodiments, the use of certain excitation frequencies can be enabled or disabled in accordance to pre-defined criteria, for example, by a respiration monitor. Additional frequency channels can also be enabled if the static component of the impedance signal is above a certain threshold, thereby potentially reducing the noise of the measurement of the variable component of the impedance. In some embodiments, configuration options can also be provided, allowing a user to enable or disable specific excitation frequencies in order to avoid undesired interactions with other devices and patient monitoring equipment.

In some embodiments, the impedance measurements may use electrode locations that are not used in standard ECG electrode placement, or not used in/for ECG measurement, for example on mid-axillary or post-axillary lines.

The more different excitation frequencies used, the more robust the respiration measurement will be against narrow-band interference that affects only a subset of the impedance measurement channels. For instance, a single pair of electrodes may be used to utilise three or more excitation frequencies to measure impedance.

In some embodiments, a plurality of electrode pairs may be used which each use a plurality of different excitation signal frequencies respectively.

In step 330, the two input signals are processed with a PCA algorithm to generate two output channels, wherein each output channel represents a respective component of the two input signals. PCA can process a set of input signals into uncorrelated output channels. In other embodiments, ICA can be used instead of PCA.

In step 340, the two output channels are analysed to determine which output channel is more probable to comprise information corresponding to the respiration rate of the subject. More specifically, the two output channels are analysed to determine which output channel is more probable to comprise an impedance signal corresponding to the subject's respiration. In other embodiments, step 340 may be performed on however many output channels are generated. After the dimensionality reduction algorithm has processed the input signals, it is likely only one of the output channels contains a respiration signal component (i.e. the 'true' respiration signal), and so by analysing the output channels to determine which is most likely to comprise information corresponding the respiration rate of the subject, the respiration rate can subsequently be estimated based on that determined output channel.

In this embodiment, step 340 comprises analysing at least one of: the variance, skewness, kurtosis, metrics of entropy, dominant frequency, and total variation of the two output channels. These metrics may aid in the determining of which output channel is most likely to comprise the respiration signal component. For example, the total variation of the respiration signal component is likely to be low compared to noise or interference.

In other words, in step 340, the output channels of the dimensionality reduction algorithm and the matrix of linear combinations can be analysed by software to find the one output channel that most likely contains the actual respiration/impedance signal. This analysis can include the variance and other statistical properties (skewness, kurtosis, metrics of entropy, statistical distribution of the sample values) of each output channel, tentative rate calculations for each output channel, and comparison with physiological respiration rate. Further, the coefficients of the linear mapping between input signals and output channels may be used.

In step 350, a respiration rate of the subject is estimated based on the output channel determined to more probably comprise information corresponding to the respiration rate of the subject. Techniques to do this are known in the art.

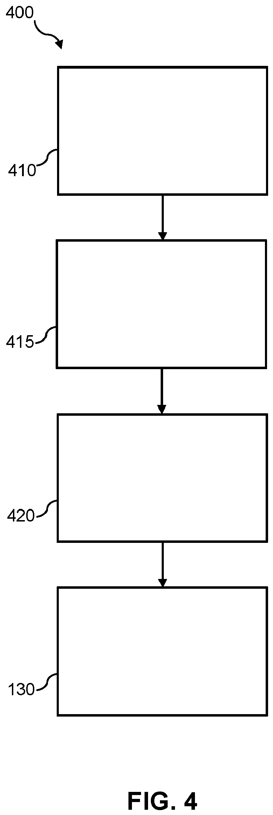

Referring now to Fig. 4, there is depicted a flow diagram of a method 400 for estimating respiration rate of a subject from a plurality of non-simultaneous input signals according to a proposed embodiment.

In step 410, a plurality of input signals is obtained, representative of thorax movement of the subject detected at different non-overlapping time periods respectively, i.e. non-simultaneously. This provides more flexibility to the method so that signals do not need to be detected simultaneously. Further, in the case of applying two different excitation frequencies with a single pair of electrodes, if the excitation frequencies are applied non-simultaneously, each impedance input signal may be acquired at a respectively higher current than if both were acquired simultaneously. This may lead to a better SNR for each input signal.

In some embodiments, obtaining the plurality of input signals may comprise using at least one pair of electrodes to apply at least two different excitation signal frequencies to the subject at different non-overlapping time periods, and to detect thoracic impedance signals for each of the different excitation signal frequencies respectively.

In step 415, the plurality of input signals is time-multiplexed to generate a plurality of time-multiplexed input signals. In some embodiments, step 415 may be skipped or replaced as needed. In other embodiments, other suitable methods for processing non-simultaneous input signals may be used instead of, or in addition to, time-multiplexing.

In step 420, the time-multiplexed input signals are processed with a dimensionality reduction algorithm to generate a plurality of output channels, wherein each output channel represents a respective component of the plurality of input signals.

Step 130 is substantially the same as has been described in relation to the method 100 in Fig. 1.

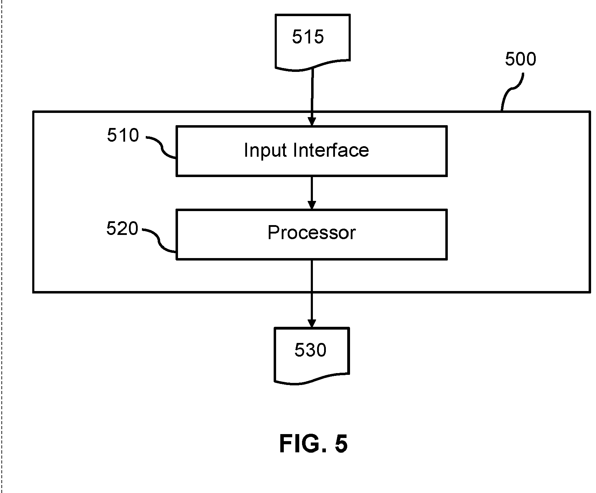

Referring now to Fig. 5, there is depicted a system 500 for estimating respiration rate of a subject according to a proposed embodiment. The system 500 comprises an input interface 510 and a processor 520.

The system 500 is configured to estimate a respiration rate of a subject by processing inputs 515. The inputs 515 comprise a plurality of input signals indicative of detected thorax movement of the subject, each of the plurality of input signals representing thorax movement detected via a respective detection arrangement. The input interface 510 obtains the inputs 515 and the processor 520 is then configured to process the plurality of input signals with a dimensionality reduction algorithm to generate a plurality of output channels, wherein each output channel represents a respective component of the plurality of input signals. The processor 520 then produces an output 530 based on at least one of the plurality of output channels. The output 530 comprises an estimated respiration rate of the subject.

In some embodiments, the system 500 may further comprise at least one pair of electrodes configured to generate the inputs 515, wherein the plurality of input signals are thoracic impedance signals.

It should be noted, many implementations of ICA operate block-wise or produce better results when operating on blocks of data instead of on a sample-by-sample basis. Some embodiments can therefore use sample-by-sample/online PCA and ICA implementations to derive a real-time wave for potential presentation on a display device. These embodiments can therefore use the presented wave to derive an estimated respiration rate, or use a block-wise PCA/ICA algorithm.

In some embodiments, the system 500 can be implemented as part of ECG measurement frontends built from discrete off-the-shelf electronic components. The system 500 may also be integrated into mixed-signal ASIC designs intended for biopotential measurements.

It should also be noted that in embodiments using impedance signals, the current invention does not depend on implementation details of an impedance measurement circuit. Impedance measurement channels can use different means to generate the respective excitation signals, such as digital-to-analog converters or microcontroller/microprocessor timer signals driving analog switches. The resulting voltage signals can then be demodulated by analog circuitry, using either IQ demodulation or demodulation with a single, phase-shifted demodulation signal. The high-frequency voltage signal can also be sampled directly using an analog-to-digital converter with sufficient sampling rate or use undersampling. The resulting digital signal can then be demodulated in software, for example, by a respiration monitor. High- and low-pass filtering of the signal can be done using analog or digital filters, as necessary and/or practical.

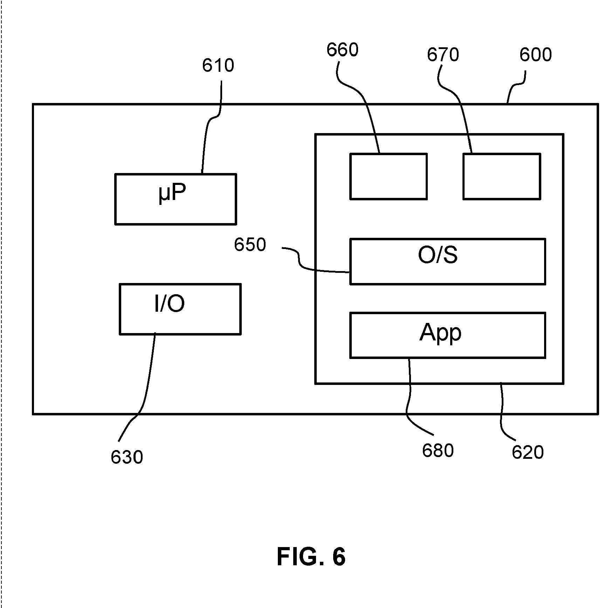

Referring now to Fig. 6, there is depicted an illustration of an example of a computer 600 within which one or more parts of an embodiment may be employed. Various operations discussed above may utilize the capabilities of the computer 600. In this regard, it is to be understood that system functional blocks can run on a single computer or may be distributed over several computers and locations (e.g., connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620 and one or more I/O devices 630 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 660, source code 670, and one or more applications 680 in accordance with exemplary embodiments. As illustrated, the application 680 comprises numerous functional components for implementing the features and operations of the exemplary embodiments. The application 680 of the computer 600 may represent various applications, computational units, logic, functional units, processes, operations, virtual entities, and/or modules in accordance with exemplary embodiments, but the application 680 is not meant to be a limitation.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed. When a source program, then the program is usually translated via a compiler (such as the compiler 660), assembler, interpreter, or the like, which may or may not be included within the memory 620, so as to operate properly in connection with the O/S 650. Furthermore, the application 680 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 630 may include input devices such as, for example but not limited to, a mouse, keyboard, scanner, microphone, camera, etc. Furthermore, the I/O devices 630 may also include output devices, for example but not limited to a printer, display, etc. Finally, the I/O devices 630 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at start-up, start the O/S 650, and support the transfer of data among the hardware devices. The BIOS is stored in some type of read-only-memory, such as ROM, PROM, EPROM, EEPROM or the like, so that the BIOS can be executed when the computer 800 is activated.

When the computer 600 is in operation, the processor 610 is configured to execute software stored within the memory 620, to communicate data to and from the memory 620, and to generally control operations of the computer 600 pursuant to the software. The application 680 and the O/S 650 are read, in whole or in part, by the processor 610, perhaps buffered within the processor 610, and then executed.

When the application 680 is implemented in software it should be noted that the application 680 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 680 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1 and 3-4 and the system of Fig. 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method (100) for estimating respiration rate of a subject, the method comprising:
obtaining a plurality of input signals (110) indicative of detected thorax movement of the subject, each of the plurality of input signals representing thorax movement detected via a respective detection arrangement;
processing the plurality of input signals (120) with a dimensionality reduction algorithm to generate a plurality of output channels, wherein each output channel represents a respective component of the plurality of input signals; and
estimating a respiration rate (130) of the subject based on at least one of the plurality of output channels.

2. The method of claim 1, wherein the plurality of input signals (515) comprises thoracic impedance signals, each of the plurality of input signals representing thorax impedance detected via a respective impendence detection arrangement.

3. The method of claim 2, wherein the plurality of input signals (515) is obtained for respective different excitation signal frequencies, and preferably wherein the different excitation signal frequencies comprise different bands of frequencies respectively.

4. The method of any prior claim, wherein obtaining the plurality of input signals (110) comprises using at least one pair of electrodes to apply at least one excitation signal frequency to the subject.

5. The method of any prior claim, wherein the plurality of input signals (110) represents thorax movement of the subject detected simultaneously.

6. The method of any prior claim, wherein obtaining the plurality of input signals (110) comprises using at least one pair of electrodes to simultaneously apply at least two different excitation signal frequencies to the subject (310, 320), and to detect thoracic impedance signals for each of the different excitation signal frequencies respectively.

7. The method of any of claims 1 to 4, wherein the plurality of input signals (515) represents thorax movement of the subject detected at different non-overlapping time periods respectively.

8. The method of claim 7, wherein obtaining the plurality of input signals (110) comprises using at least one pair of electrodes to apply at least two different excitation signal frequencies to the subject at different non-overlapping time periods, and to detect thoracic impedance signals for each of the different excitation signal frequencies respectively.

9. The method of any prior claim, wherein the dimensionality reduction algorithm comprises at least one of a principal component analysis algorithm and an independent component analysis algorithm.

10. The method of any prior claim, wherein estimating a respiration rate (130) of the subject based on at least one of the plurality of output channels comprises:
analysing the plurality of output channels (340) to determine which output channel is more probable to comprise information corresponding to the respiration rate of the subject; and
estimating a respiration rate (350) of the subject based on the determined output channel.

11. The method of claim 10, wherein analysing the plurality of output channels (340) to determine which output channel is more probable to comprise information corresponding to the respiration rate of the subject comprises:
analysing at least one of: the variance, skewness, kurtosis, metrics of entropy, dominant frequency, and total variation of the plurality of output channels.

12. The method of any prior claim, wherein obtaining the plurality of input signals (110) comprises using at least one of:
at least one strain gauge attached the thorax, and at least one accelerometer attached to the thorax.

13. A computer program (680) comprising code means for implementing the method of any preceding claim when said program is run on a processing system.

14. A system (500) for estimating respiration rate of a subject, the system comprising:
an input interface (510) configured to:
obtain a plurality of input signals (515) indicative of detected thorax movement of the subject, each of the plurality of input signals representing thorax movement detected via a respective detection arrangement; and
a processor (520) configured to:
process the plurality of input signals (120) with a dimensionality reduction algorithm to generate a plurality of output channels, wherein each output channel represents a respective component of the plurality of input signals; and
estimate a respiration rate (130) of the subject based on at least one of the plurality of output channels.

15. The system of claim 14, wherein the system further comprises:
at least one pair of electrodes configured to generate the plurality of input signals (515), wherein the plurality of input signals are thoracic impedance signals.
